(19) Europäisches Patentamt
European Patent Office
Office européen des brevets

(11) EP 1 497 246 B1

(12) **EUROPÄISCHE PATENTSCHRIFT**

(45) Veröffentlichungstag und Bekanntmachung des Hinweises auf die Patenterteilung:
**04.03.2009 Patentblatt 2009/10**

(21) Anmeldenummer: **03712078.9**

(22) Anmeldetag: **22.03.2003**

(51) Int Cl.:
***C07C 29/151*** (2006.01) ***C07C 31/04*** (2006.01)
***C01B 3/02*** (2006.01) ***C01C 1/04*** (2006.01)

(86) Internationale Anmeldenummer:
**PCT/EP2003/003011**

(87) Internationale Veröffentlichungsnummer:
**WO 2003/106393 (24.12.2003 Gazette 2003/52)**

(54) **ANLAGE UND VERFAHREN ZUR ERZEUGUNG VON SYNTHESEGASEN AUS ERDGAS**

INSTALLATION AND METHOD FOR PRODUCING AND DISAGGREGATING SYNTHESIS GASES FROM NATURAL GAS

INSTALLATION ET PROCEDE POUR PRODUIRE ET SEPARER DES GAZ DE SYNTHESE A PARTIR DE GAZ NATUREL

(84) Benannte Vertragsstaaten:
**DE DK FR GB IT**

(30) Priorität: **13.06.2002 DE 10226209**

(43) Veröffentlichungstag der Anmeldung:
**19.01.2005 Patentblatt 2005/03**

(73) Patentinhaber: **Lurgi AG**
**60295 Frankfurt am Main (DE)**

(72) Erfinder:
- **DAVEY, William**
  **60439 Frankfurt am Main (DE)**
- **MEYER, Manfred**
  **61381 Friedrichsdorf (DE)**
- **HOFMOCKEL, Jürgen**
  **60314 Frankfurt am Main (DE)**

(74) Vertreter: **Meyer-Dulheuer, Karl-Hermann**
**Dr. Meyer- Dulheuer & Partner**
**Patentanwaltskanzlei**
**Barckhausstrasse 12-16**
**60325 Frankfurt am Main (DE)**

(56) Entgegenhaltungen:
**BE-A- 865 319** **DE-A- 2 705 673**
**DE-A- 2 904 008** **US-A- 4 110 359**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Gegenstand der Erfindung ist eine Anlage und ein Verfahren zur gleichzeitigen Herstellung von Synthesegasen wie Methanol-Synthesegas, Ammoniak-Synthesegas, Kohlenmonoxid und Kohlendioxid durch Zerlegung eines aus Erdgas gewonnenen Gasgemisches.

Es ist bekannt, zur Herstellung von Methanol, Ammoniak, reinem Kohlenmonoxid, Kohlendioxid und anderen Synthesegasen, Produktionsanlagen zu errichten, in denen in der Regel jeweils nur eines der vorstehend genannten Gase hergestellt werden kann [(2) und (3)]. Lediglich Verfahren zur gleichzeitigen Herstellung von Methanol und Ammoniak sind aus der DE-OS 33 36 649, der japanischen Patentanmeldung JP 200 006 3115 und dem europäischen Patent 0 853 608 bereits bekannt geworden. Ein hierfür geeigneter, technisch wichtiger Weg beinhaltet die Umwandlung eines natürlichen Erdgases in ein Synthesegas, das als Hauptbestandteile Kohlenmonoxid, Kohlendioxid und Wasserstoff enthält. Verfahren zur Herstellung von Synthesegas sind z.B. in der DE-OS 33 45 064 und der europäischen Patentanmeldung EP-A-0 999 178 beschrieben.

Aus wirtschaftlichen Gründen wäre es nun allerdings sehr vorteilhaft, wenn es gelänge, in einer einzigen Produktionsanlage eine so vollständige Zerlegung der Bestandteile eines Synthesegases durchzuführen, dass in einem einzigen Produktionsstrang Methanol-Synthesegas, Ammoniak-Synthesegas, Kohlenmonoxid und Kohlendioxid in großer Reinheit gewonnen werden können, so dass sie unmittelbar für weitere chemische Synthesen einsetzbar sind. Eine derartige kombinierte Gaszerlegungsanlage wäre nicht nur wegen der damit erreichbaren Einsparungen durch erhöhte Produktionsmengen, sondern auch deshalb besonders wirtschaftlich, weil mehrere Anlageneinheiten im Gegensatz zu mehreren, jeweils nur auf ein einzelnes Produkt ausgerichteten Anlagen nur in einer einzigen Ausfertigung benötigt werden würden. Besonders wirtschaftlich könnte eine derartige Anlage dann sein, wenn sie so flexibel ausgestaltet werden könnte, dass die Mengen der in einer derartigen Anlage gewonnenen unterschiedlichen Gase dem jeweiligen Bedarf angepasst werden können.

Es wurde nun gefunden, dass diese Anforderungen durch eine Anlage zur gleichzeitigen Herstellung von Methanol-Synthesegas, Ammoniak-Synthesegas, Kohlenmonoxid und Kohlendioxid aus Erdgas erfüllt werden, wenn in einem einzigen Anlagenstrang hintereinander in einer Reihe die folgenden Anlageneinheiten angeordnet sind:

- ein erster Reaktor A zur Durchführung einer katalytischen Teiloxidation des Erdgases mit Zuleitungen für Erdgas, Wasserdampf und Sauerstoff, wobei in dem Reaktor A unter Zuführung von Sauerstoff das Erdgas in ein Synthesegasgemisch aus Kohlenmonoxid, Kohlendioxid, Wasserstoff und Wasserdampf umgewandelt wird,

- ein zweiter Reaktor B, in dem die Umwandlung von Kohlenmonoxid in Kohlendioxid gesteuert wird,

- ein Absorber D, der zur Absorption von Kohlendioxid und zur Gewinnung des zur Methanolsynthese verwendeten Kohlenmonoxid-Wasserstoffgemisches dient,

- ein Kälteabscheider E, in dem durch Zuleitung von flüssigem Stickstoff Ammoniaksynthesegas gewonnen und gleichzeitig Kohlenmonoxid, Argon und Methan abgetrennt werden, wobei der Kälteabscheider E Zuleitungen für das von Kohlendioxyd befreite Synthesegas sowie für flüssigen Stickstoff und Ableitungen für das Ammoniak-Synthesegas, für reines Kohlenmonoxid, für ein Methan, Argon und Kohlenmonoxid enthaltendes Brenngas und für eine Kohlenmonoxid und Wasserstoff enthaltende Flüssigkeit aufweist.

Der Reaktionsablauf ist in der beiliegenden Fig.1 schematisch dargestellt.

Der erste Reaktor A dient zur Gewinnung von Synthesegas und ermöglicht die Entschwefelung des eingesetzten Gasgemisches, seine Sättigung mit Wasserdampf, das Erwärmen in einem Erhitzer unter katalytischer Zersetzung längerkettiger Kohlenwasserstoffe zu Methan, die durch partielle Oxidation mit Sauerstoff sowie ein Abkühlen des Gases unter Erzeugung von Dampf. Eine derartige Anlageneinheit, die auch als CPox-Reaktor (catalytic partial oxidation) bezeichnet wird, wird im Anlagenbau häufig eingesetzt und ist in der Literatur (1) beschrieben. Es handelt sich dabei um ein zylindrisches Gefäß mit vertikal angeordneten gewölbten Wänden. Ein Brenner oder Mischer ist im oberen Teil des Gefäßes vorgesehen, in welches ein mit Wasserdampf gemischtes natürliches Erdgas, Dampf und Sauerstoff durch separate Zuleitungen eingeführt werden. Der Brenner oder Mischer fördert die kräftige Vermischung dieser drei Gasströme im oberen Teil des Gefäßes, in dem der Hauptanteil der partiellen Oxidation sehr schnell stattfindet. Die heißen Gase werden dann über einen im unteren Teil des Gefäßes vorhandenen Katalysator geleitet, wo die Umwandlung des Erdgases vervollständigt wird. Die katalytische Teiloxidation lässt sich durch die folgenden chemischen Reaktionsgleichungen charakterisieren:

$$CH_4 + H_2O \rightarrow CO + 3\,H_2 \quad (1)$$

| | |
|---|---|
| $CH_4 + 2 O_2 \longrightarrow CO_2 + 2 H_2O$ | (2) |

| | |
|---|---|
| $2 CO + O_2 \longrightarrow 2 CO_2$ | (3) |

| | |
|---|---|
| $2 H_2 + O_2 \longrightarrow 2 H_2O$ | (4) |

| | |
|---|---|
| $CO_2 + H_2 \longrightarrow CO + H_2O$ | (5) |

Wasserdampf wird dem Reaktor A in einer solchen Menge zugeleitet, dass ein Molverhältnis von Wasserdampf zu den nicht oxidierten Kohlenwasserstoffen von 1,4 zu 3,0, vorzugsweise von 1,7 eingestellt wird. Sauerstoff wird dem Reaktor A in einer Menge zugeleitet, dass das Molverhältnis von Sauerstoff zu den nicht oxidierten Kohlenwasserstoffen zwischen 0,45 und 0,7, vorzugsweise bei 0,52 liegt. Die genaue Menge an Sauerstoff wird in der Praxis dadurch kontrolliert, dass man die Ausgangstemperatur das Gasgemisches aus dem Reaktor A auf Temperaturen zwischen 900 und 1050 °C, im allgemeinen von 950°C einstellt. Die Reinheit des Sauerstoffs, der von der Luftzerlegungsanlage F (siehe Fig. 1) geliefert wird, beträgt im allgemeinen zwischen 90 und 99,5%, liegt aber normalerweise bei 99,5 %. Der im Reaktor A verwendete Katalysator ist ein Nickeloxidkatalysator, z.B. ein Katalysator der Typen G-31 E, G-90LDP oder G-90B, die von der Süd-Chemie AG, München, bezogen werden können. Die Umwandlung des Erdgases in ein Synthesegas erfolgt bei einem Druck von 20 bis 100 bar, vorzugsweise bei einem Druck von etwa 40 bar.

Der Reaktor A ist mit einem zweiten Reaktor B verbunden, in dem die Bildung von Kohlendioxid aus Kohlenmonoxid unter gleichzeitiger Erzeugung von Wasserstoff gesteuert werden kann. Der Reaktor B weist aber auch eine Umleitung [3] auf, durch die das im ersten Reaktor A gewonnene Synthesegasgemisch ganz oder teilweise an dem Reaktor B vorbeigeleitet und so dessen Konvertierungsgrad gesteuert werden kann. In dem Reaktor B findet die Oxidation des Kohlenmonoxids zum Kohlendioxid als ein ein- oder zweistufiges Verfahren mit dazwischen eingeschobener Kühlung in Anwesenheit von Hochtemperaturkatalysatoren statt.

Falls kein oder nur ein geringer Bedarf an Kohlendioxid besteht, wird das aus dem Reaktor A gewonnene Synthesegas am Reaktor B vorbeigeleitet und gelangt dann direkt über die Leitung [4] in einen Verdichter C, in dem das entstandene Gasgemisch komprimiert werden kann. Aufgabe dieses Verdichters C ist es, das im Reaktor A gebildete Gas auf einen Druck zwischen 60 und 100 bar, im allgemeinen auf einen Druck von 80 bar zu bringen. Liegt jedoch der Druck des aus dem Reaktor A entnommenen Gases bereits über 40 bar, kann auf den Einsatz eines Verdichters verzichtet werden. Der hier eingesetzte Verdichter ist eine bekannte Vorrichtung, wie sie in vielen chemischen Anlagen üblicherweise eingesetzt werden.

Vom Kompressor C wird das Gasgemisch über die Leitung [5] in den Absorber D geleitet, in dem das Kohlendioxid aus dem Gasgemisch entfernt wird. Das kann entweder auf physikalischem oder auf chemischem Wege erfolgen. Bei einer physikalischen Gaswäsche wird das Kohlendioxid durch kaltes Methanol oder kalten Glykolether absorbiert. Eine chemische Wäsche erfolgt die Absorption, vorzugsweise durch ein Alkanolamin, Natriumkarbonat oder eine andere alkalische Substanz. Vorzugsweise weist der Absorber D zwei Reaktionsstufen auf, wobei in der ersten Reaktionsstufe eine grobe Abtrennung des Kohlendioxids bis zu einer molaren Konzentration zwischen 1 und 10 Gewichtsprozent, berechnet auf Basis des trockenen Gases, vorzugsweise aber eine Entfernung bis zu einer Menge von 2,2 Gewichtsprozent erfolgt. In der zweiten Absorptionsstufe wird dann das übrige Kohlendioxid bis zu einer molaren Konzentration von weniger als 50 ppm, vorzugsweise weniger als 10 ppm, entfernt. Der Absorber D beinhaltet außerdem eine Vorrichtung zur kontrollierten Reduktion des Gasdruckes des Absorptionsmittels, um dadurch Kohlendioxid zurückgewinnen zu können. Außerdem enthält der Absorber D auch Vorrichtungen zur Regeneration des Absorptionsmittels durch Anwendung von Hitze, Vorrichtungen zur Aufrechterhaltung einer gleich bleibenden Zusammensetzung des Absorptionsmittels sowie für die Einstellung des Gasdruckes des Lösungsmittels auf den Verfahrensdruck. Das so wieder gewonnene Kohlendioxid kann ganz oder teilweise für anschließende Synthesen verwendet werden, z.B. für die Herstellung von

Harnstoff. Überschüssiges Kohlendioxid wird in die Atmosphäre abgelassen. Verschiedene andere Verfahren zur Entfernung von Kohlendioxid sind in den Literaturstellen (2), (3) und (4) beschrieben.

Das nunmehr vom Kohlendioxid befreite Gasgemisch wird dann über die Leitung [7] in den Kälteabscheider E eingeleitet, in dem eine teilweise Kondensation und Abtrennung von Kohlenmonoxid und Wasserstoff durch Einleiten von flüssigem Stickstoff erfolgt. Dieses Verfahren ist in der gleichzeitig eingereichten deutschen Patentanmeldung DE 102 26 210 A1 im einzelnen beschrieben. Dadurch wird ein aus Kohlenmonoxid und Wasserstoff bestehendes Methanol-Synthesegas gewonnen. Die Reinheit des im Kälteabscheider E gewonnenen Kohlenmonoxids kann durch eine Methanwäsche weiter verbessert werden.

Das im Kälteabscheider E gewonnene Kohlenmonoxid kann aber auch in eine Anlage zur Herstellung von Essigsäure durch Carbonylierung von Methanol eingeleitet werden.

Verunreinigungen durch Methan oder Argon werden durch die Stickstoffwäsche im Kälteabscheider E ebenfalls entfernt und können als Brenngas zur Hitzeerzeugung im Reaktor A verwertet werden.

Im Kälteabscheider E wird das Gas auf eine Temperatur zwischen -200°C und -150 °C abgekühlt. Bei dieser Temperatur wird das Gas in einer oder mehreren Verdampfungstrommeln einer Flash-Verdampfung unterworfen und dadurch Wasserstoff vom Kohlenmonoxid getrennt. Bei den Flash-Verdampfungen wird zunächst ein an Kohlenmonoxid reicher flüssiger Wasserstoff gebildet. Gasförmiges Kohlenmonoxidgas wird zur Reinigung mit flüssigem Kohlenmonoxid gewaschen, um Methan zu entfernen, und dann wieder auf Zimmertemperatur erwärmt. Der Wasserstoff wird über eine zweite Waschsäule geleitet wo er mit flüssigem Stickstoff gewaschen wird, um Spuren von Kohlenmonoxid, Argon und Methan zu entfernen. Das molare Verhältnis von Wasserstoff zu Stickstoff wird dann auf einen Wert von 3:1 eingestellt, um ein für die Ammoniaksynthese geeignetes Gasgemisch zu gewinnen.

Der Kälteabscheider E enthält auch ein Molekularsieb, um schon vor der Kältetrennung Spuren von Kohlendioxid abzuscheiden und so ein kohlendioxidfreies Synthesegas zu gewinnen. Auch der Kälteabscheider E ist eine bekannte Anlageneinheit und in der Literaturstelle (5) ausführlich beschrieben.

Die in Fig. 1 dargestellte Anlageneinheit F ist eine übliche Luftzerlegungsanlage, die einen Sauerstoffstrom mit einer Reinheit zwischen 90 und 99,5 % liefert. Die Anlageneinheit F stellt außerdem Stickstoff mit einer Reinheit von mehr als 99,995 % her.

Die in der erfindungsgemäßen Anlage nach den vorstehend beschriebenen Verfahren gewonnenen Gase fallen in so großer Reinheit an, dass sie für chemische Folgensynthesen verwendet werden können.

Die Leistungsfähigkeit der erfindungsgemäßen Anlage, die auf die nachfolgend genannten Produktionsmengen ausgelegt ist, und das darin durchzuführende Verfahren zur Zerlegung eines Synthesegases wird durch das folgende Beispiel unterstrichen:

a) Es sollen 4000 Tonnen pro Tag Methanol hergestellt werden, von dem ein Teil zur Herstellung von Essigsäure verwendet wird. Die Synthese von Methanol erfordert eine Zusammensetzung des Synthesegases mit einer stöchiometrischen Zahl $S_n$ von 2,05, eine Kohlendioxidkonzentration im Bereich zwischen 2 und 3 %, eine Stickstoffkonzentration von weniger als 0,5%. Die stöchiometrische Zahl ($S_n$) wird nach der folgenden Formel berechnet:

$$S_n = \frac{([H_2]-[CO_2])}{([CO_2]+[CO])}$$

Hierbei bedeuten $[H_2]$ $[CO_2]$ und $[CO_2]$ $[CO]$ die molaren Konzentrationen von Wasserstoff, Kohlendioxid und Kohlenmonoxid im Synthesegas;

b) Gleichzeitig können aus der gleichen Anlage Synthesegase für 1200 Tonnen Essigsäure pro Tag gewonnen werden. Die Herstellung von Essigsäure erfordert Methanol und Kohlenmonoxid mit einer Reinheit von wenigstens 98 %;

c) Außerdem können aus der gleichen Anlage Synthesegase für 4000 Tonnen Ammoniak pro Tag zur Verfügung gestellt werden, von dem ein Teil zur Herstellung von Harnstoff verwendet wird. Die Herstellung von Ammoniak erfordert eine Mischung von Wasserstoff und Stickstoff im molaren Verhältnis von 3:1, wobei das Gasgemisch weniger als 10 ppm Sauerstoff enthalten muss.

d) Schließlich können aus der gleichen Anlage auch noch Synthesegase für 6270 Tonnen Harnstoff pro Tag gewonnen werden. Die Herstellung von Harnstoff erfordert reinen Ammoniak sowie Kohlendioxid in einer Reinheit über 98,5%.

Diese Anforderungen können bei folgender Vorgehensweise erfüllt werden, wobei die Zusammensetzung der einzelnen Gasströme in der Tabelle 1 angegeben ist:

1. Das aus dem Erdgas hergestellte rohe Synthesegas wird in dem Reaktor A hergestellt und auf einen Druck von etwa 45 bar eingestellt. Es verlässt den Reaktor A mit der Zusammensetzung [2];

2. Etwa 82% des rohen Synthesegases des Reaktors A werden als Gasstrom [3] um den Reaktor B herumgeleitet, während 18% des rohen Synthesegases im Reaktor B einer gesteuerten Umwandlung von Kohlenmonoxid in Kohlendioxid unterworfen werden. Der aus dem Reaktor B austretende Gasstrom wird dann mit dem Gasstrom [3] zum Gasstrom [4] vereinigt;

3. Der gekühlte und kondensierte Gasstrom [4] im Verdichter C wird auf einen Druck von etwa 80 bar komprimiert;

4. Das komprimierte Gas wird in den Absorber D eingespeist, aus dem etwa 43% des Synthesegases abgezogen wird, wenn das Kohlendioxid-Absorptionsmittel einen mittleren Sättigungsgrad erreicht und die Kohlendioxidkonzentration auf etwa 2,2% vermindert ist. Das Gas hat dann die Zusammensetzung des Gasstroms [6]. Das restliche Gas wird in der Kohlendioxid-Feinwäsche einer zweiten Absorption unterworfen und dabei eine Kohlendioxidkonzentration von weniger als 10 ppm hergestellt. Dieses Gas wird als Gasstrom [7] in den Kälteabscheider E geleitet.

5. Im Kälteabscheider E wird aus dem Synthesegas Kohlenmonoxid abgeschieden, das als Gasstrom [10] zur Essigsäuresynthese oder zur Ammoniaksynthese als Gasstrom [11] sowie als Restgasstrom (Gasstrom [8]) verwertet wird, wobei der Gasstrom 8 mit dem Methanolsynthesegas im Gasstrom [9] vereinigt wird. Verunreinigungen des Synthesegases wie Methan, Argon und Kohlenmonoxid werden als Brenngas verwertet und dem Brenner des Reaktors A zugeleitet;

6. Das aus dem Absorber D gewonnene Kohlendioxid wird über den Stoffstrom [15] zur Harnstoffsynthese verwendet.

Die vorstehend beschriebene Zerlegung des Synthesegases in mehrer Einzelfraktionen in einer einzigen Anlage ist nur ein Beispiel für die praktisch unbegrenzte Möglichkeit der Bereitstellung von für spezifische chemische Synthesen erforderliche Gaszusammensetzungen durch Kombination der in der erfindungsgemäßen Anlage erhaltenen Anlageeinheiten und chemischen Umwandlungsverfahren. Durch entsprechende Abwandlungen und Änderungen der einzelnen Anlageeinheiten und Verfahrensschritte können in einer einzigen Anlage aus Erdgas auch spezielle Gasgemische für andere wichtige Synthesen, wie die Fischer-Tropsch-Synthese, die Oxo-Alkohol-Synthese, die Ethylen-Glykol-Synthese und andere Verfahren gewonnen werden.

**Literaturverzeichnis:**

1. Hermann Göhna, "Concepts for Modern Methanol Plants". Proceedings of the 1997 World Methanol Conference, Tampa, Florida, USA (December 1997)

2. "Gas Production", Ullmans's Encyclopedia of Industrial Chemistry, Vol. A12, VCH Verlagsgesellschaft mbh (1989)

3. Max App "Ammonia, Methanol, Hydrogen, Carbon Monoxide, Modem Production Technologies". British Sulphur Publishing - a Division of CRU Publishing Ltd., 31 Mount Pleasant, London WC1X 0AD. ISBN 1 873387 26 1 (published 1997)

4. Emil Supp "How to produce Methanol from Coal". Springer-Verlag (1990)

5. W L E Davey "Cold Box for the Production of Multiple Products from a Stream of Syngas". German Patent Application No. ? (2002) (L1 P13)

**Patentansprüche**

1. Anlage zur gleichzeitigen Herstellung von Methanol-Synthesegas, Ammoniak-Synthesegas, Kohlenmonoxid und Kohlendioxid aus Erdgas, **dadurch gekennzeichnet, dass** in einem einzigen Anlagenstrang hintereinander in einer

Reihe mehrere Anlageneinheiten angeordnet sind umfassend

- einen ersten Reaktor A zur Durchführung einer katalytischen Teiloxidation des Erdgases mit Zuleitungen für Erdgas, Wasserdampf und Sauerstoff, wobei in dem Reaktor A unter Zuführung von Sauerstoff das Erdgas in ein Synthesegasgemisch aus Kohlenmonoxid, Kohlendioxid, Wasserstoff und Wasserdampf umgewandelt wird,
- einen zweiten Reaktor B, in dem die Umwandlung von Kohlenmonoxid in Kohlendioxid gesteuert wird,
- einen Absorber D, der zur Absorption von Kohlendioxid und zur Gewinnung des zur Methanolsynthese verwendeten Kohlenmonoxid-Wasserstoffgemisches dient,
- einen Kälteabscheider E, in dem durch Zuleitung von flüssigem Stickstoff Ammoniaksynthesegas gewonnen und gleichzeitig Kohlenmonoxid, Argon und Methan abgetrennt werden, wobei der Kälteabscheider E Zuleitungen für das von Kohlendioxyd befreite Synthesegas sowie für flüssigen Stickstoff und Ableitungen für das Ammoniak-Synthesegas, für reines Kohlenmonoxid, für ein Methan, Argon und Kohlenmonoxid enthaltendes Brenngas und für eine Kohlenmonoxid und Wasserstoff enthaltende Flüssigkeit aufweist.

**2.** Anlage nach Anspruch 1, **dadurch gekennzeichnet, dass** ein Verdichter C vorgesehen ist, in dem die in den Reaktoren A und B entstandenen Gase komprimiert werden können.

**3.** Anlage nach den Ansprüchen 1 oder 2, **dadurch gekennzeichnet, dass** der zweite Reaktor eine Umleitung [3] aufweist, durch die das im Reaktor A gewonnene Synthesegasgemisch ganz oder teilweise am Reaktor B vorbei geleitet und so dessen Oxidationsgrad, insbesondere die Bildung von Kohlendioxid aus Kohlenmonoxid, gesteuert werden kann.

**4.** Anlage nach den Ansprüchen 2 bis 3, **dadurch gekennzeichnet, dass** ein Verdichter C vorgesehen ist, durch den der Gasdruck auf einen Wert eingestellt werden kann, der die physikalische oder chemische Absorption des Kohlendioxids im Absorber D sicherstellt.

**5.** Anlage nach den Ansprüchen 1 bis 4, **dadurch gekennzeichnet, dass** der Absorber D eine Zuleitung für das Synthesegasgemisch und Ableitungen für das aus Kohlenmonoxid und Wasserstoff bestehende Methanol-Synthesegas, für Kohlendioxid und für das von Kohlendioxid befreite Synthesegas sowie für andere Synthesen geeignete Gase mit entsprechender Zusammensetzung aufweist.

**6.** Verfahren zur gleichzeitigen Herstellung von Methanol-Synthesegas, Ammoniak-Synthesegas, Kohlenmonoxid und Kohlendioxid aus Erdgas durch Zugabe von Wasserdampf und Sauerstoff in einer Anlage nach den Ansprüchen 1 bis 5, **dadurch gekennzeichnet, dass** folgende Schritte ausgeführt werden:

a) katalytische Teiloxidation des Erdgases in einem ersten Reaktor A mit Zuleitungen für Erdgas, Wasserdampf und Sauerstoff, wobei in dem ersten Reaktor A unter Zuführung von Sauerstoff das Erdgas in ein Synthesegasgemisch aus Kohlenmonoxid, Kohlendioxid, Wasserstoff und Wasserdampf umgewandelt wird und im ersten Reaktor A das Molverhältnis von Wasserdampf zu den nicht-oxidierten Kohlenwasserstoffen von 1,5 bis zu 3,0, vorzugsweise 1,7, beträgt.
b) Steuerung der Umwandlung von Kohlenmonoxid in Kohlendioxid in einem zweiten Reaktor B,
c) Absorption von Kohlendioxid und Gewinnung des zur Methanolsynthese verwendeten Kohlenmonoxid-Wasserstoffgemisches in einem Absorber D und
d) Gewinnung von Ammoniaksynthesegas und gleichzeitige Abtrennung von Kohlenmonoxid, Argon und Methan durch Zuleitung von flüssigem Stickstoff und einen Kälteabscheider E, wobei der Kälteabscheider E Zuleitungen für das von Kohlendioxyd befreite Synthesegas sowie für flüssigen Stickstoff und Ableitungen für das Ammoniak-Synthesegas, für reines Kohlenmonoxid, für ein Methan, Argon und Kohlenmonoxid enthaltendes Brenngas und für eine Kohlenmonoxid und Wasserstoff enthaltende Flüssigkeit aufweist.

**7.** Verfahren nach Anspruch 6, **dadurch gekennzeichnet, dass** im ersten Reaktor das Molverhältnis von Sauerstoff zu den nicht-oxidierten Kohlenwasserstoffen 0,45 bis 0,7, vorzugsweise 0,52 beträgt.

**8.** Verfahren nach den Ansprüchen 6 und 7, **dadurch gekennzeichnet, dass** zur Umwandlung des Erdgases in ein Synthesegas ein Nickeloxid enthaltender Katalysator eingesetzt wird.

**9.** Verfahren nach den Ansprüchen 6 bis 8, **dadurch gekennzeichnet, dass** zur Umwandlung des Erdgases in ein Synthesegas nach den Ansprüchen 8 bis 10 ein Druck von 25 bis 100 bar, vorzugsweise ein Druck von etwa 40 bar, eingesetzt wird.

10. Verfahren nach den Ansprüchen 6 bis 9, **dadurch gekennzeichnet, dass** zur Oxidation des Kohlenmonoxids zu Kohlendioxid im zweiten Reaktor B ein ein- oder zweistufiges Verfahren mit dazwischen eingeschobener Kühlung in Anwesenheit von Hochtemperatur-Katalysatoren durchgeführt wird.

11. Verfahren nach den Ansprüchen 6 bis 10, **dadurch gekennzeichnet, dass** das in den Reaktoren A und B erzeugte Synthesegas durch den Verdichter C auf einen Druck von 60 bis 100 bar, vorzugsweise auf einen Druck von 80 bar eingestellt wird.

12. Verfahren nach den Ansprüchen 6 bis 11, **dadurch gekennzeichnet, dass** im Absorber D das Kohlendioxid aus dem Synthesegas in einem oder mehreren Verfahrensschritten auf chemischem oder physikalischem Weg abgeschieden wird.

13. Verfahren nach den Ansprüchen 6 bis 12, **dadurch gekennzeichnet, dass** das Synthesegas im Abscheider durch Einleiten von flüssigem Stickstoff auf Temperaturen von -150°C bis -200°C abgekühlt und **dadurch** Methan, Argon und Kohlenmonoxid abgetrennt werden und das verbliebene Wasserstoffgas mit Stickstoff im Molverhältnis 3:1 zur Gewinnung von Ammoniaksynthesegas vermischt wird.

## Claims

1. An installation for the simultaneous production of methanol synthesis gas, ammonia synthesis gas, carbon monoxide, and carbon dioxide from natural gas, **characterized in that** a plurality of installation units are arranged in series one behind the other in a single train of the installation, the installation comprising

    • a first reactor A for performing a catalytic partial oxidation of the natural gas having supply pipes for natural gas, water vapor, and oxygen, the natural gas being converted to a synthesis gas mixture of carbon monoxide, carbon dioxide, hydrogen, and water vapor in reactor A with the addition of oxygen;
    • a second reactor B in which the conversion of carbon monoxide to carbon dioxide is controlled;
    • an absorber D, which is used for absorbing carbon dioxide and for producing the carbon monoxide/hydrogen mixture used for the synthesis of methanol;
    • a cold separator E in which, by supplying liquid nitrogen, ammonia synthesis gas is obtained and, simultaneously, carbon monoxide, argon, and methane are removed, cold separator E having supply pipes for the synthesis gas freed of carbon dioxide and for liquid nitrogen and discharge pipes for the ammonia synthesis gas, for pure carbon monoxide, for a combustible gas comprising methane, argon, and carbon monoxide, and for a liquid comprising carbon monoxide and hydrogen.

2. The installation according to claim 1, **characterized in that** a compressor C is provided, in which the gases produced in reactors A and B can be compressed.

3. The installation according to claim 1 or claim 2, **characterized in that** the second reactor has a bypass [3] through which the synthesis gas mixture obtained in reactor A can completely or partially bypass reactor B and thus the degree of oxidation thereof, in particular the formation of carbon dioxide from carbon monoxide, can be controlled.

4. The installation according to claims 2 to 3, **characterized in that** a compressor C is provided, which allows the adjustment of the gas pressure to a value that guarantees the physical or chemical absorption of the carbon dioxide in absorber D.

5. The installation according to claims 1 to 4, **characterized in that** the absorber D has a supply pipe for the synthesis gas mixture and discharge pipes for the methanol synthesis gas consisting of carbon monoxide and hydrogen, for carbon dioxide, and for the synthesis gas freed of carbon dioxide, and for gases suitable for other syntheses having the appropriate composition.

6. A method for the simultaneous production of methanol synthesis gas, ammonia synthesis gas, carbon monoxide, and carbon dioxide from natural gas by adding water vapor and oxygen in an installation according to claims 1 to 5, **characterized in that** the following steps are performed:

    a) catalytic partial oxidation of the natural gas in a first reactor A having supply pipes for natural gas, water vapor, and oxygen, the natural gas being converted to a synthesis gas mixture of carbon monoxide, carbon

dioxide, hydrogen, and water vapor with the addition of oxygen in reactor A and the molar ratio of water vapor to non-oxidized hydrocarbons in the first reactor A being from 1.5 to 3.0, preferably 1.7;
b) controlling the conversion of carbon monoxide to carbon dioxide in a second reactor B;
c) absorbing carbon dioxide and obtaining the carbon monoxide/hydrogen mixture used for methanol synthesis in an absorber D; and
d) obtaining ammonia synthesis gas and simultaneously removing carbon monoxide, argon, and methane by supplying liquid nitrogen in a cold separator E, cold separator E having supply pipes for the synthesis gas freed of carbon dioxide and for liquid nitrogen and discharge pipes for the ammonia synthesis gas, for pure carbon monoxide, for a combustible gas comprising methane, argon, and carbon monoxide, and for a liquid comprising carbon monoxide and hydrogen.

**7.** The method according to claim 6, **characterized in that** in the first reactor, the molar ratio of oxygen to non-oxidized hydrocarbons is 0.45 to 0.7, preferably 0.52.

**8.** The method according to claims 6 and 7, **characterized in that** a catalyst containing nickel oxide is used for the conversion of natural gas to a synthesis gas.

**9.** The method according to claims 6 to 8, **characterized in that** for the conversion of the natural gas to a synthesis gas according to claims 8 to 10, a pressure of 25 to 100 bar, preferably a pressure of approximately 40 bar, is used.

**10.** The method according to claims 6 to 9, **characterized in that** for the oxidation of carbon monoxide to carbon dioxide in second reactor B, a one-step or a two-step process with in-between cooling in the absence of high-temperature catalysts is performed.

**11.** The method according to claims 6 to 10, **characterized in that** the synthesis gas produced in reactors A and B is adjusted to a pressure of 60 to 100 bar, preferably to a pressure of 80 bar, by means of compressor C.

**12.** The method according to claims 6 to 11, **characterized in that** in absorber D, carbon dioxide is removed from the synthesis gas in one or more process steps by chemical or physical means.

**13.** The method according to claims 6 to 12, **characterized in that** in the separator, the synthesis gas is cooled to temperatures of -150 °C to -200 °C by introducing liquid nitrogen and methane, argon, and carbon monoxide are thereby removed, and the remaining hydrogen gas is mixed with nitrogen in a molar ratio of 3:1 to produce ammonia synthesis gas.

**Revendications**

**1.** Installation pour la production simultanée de gaz de synthèse du méthanol, de gaz de synthèse de l'ammoniac, de monoxyde de carbone et de dioxyde de carbone à partir de gaz naturel, **caractérisée en ce que** dans un seul tronçon de l'installation sont disposées les unes derrière les autres, dans une rangée, plusieurs unités de l'installation, comprenant

• un premier réacteur A pour réaliser une oxydation catalytique partielle du gaz naturel avec des conduits d'arrivée pour du gaz naturel, de la vapeur d'eau et de l'oxygène, le gaz naturel étant transformé dans le réacteur A, avec alimentation d'oxygène en un mélange de gaz de synthèse de monoxyde de carbone, de dioxyde de carbone, d'hydrogène et de vapeur d'eau,
• un deuxième réacteur B, dans lequel est commandée la transformation de monoxyde de carbone en dioxyde de carbone,
• un absorbeur D, servant à l'absorption de dioxyde de carbone et à la préparation du mélange monoxyde de carbone/hydrogène utilisé pour la synthèse du méthanol,
• un séparateur à froid E, dans lequel, par arrivée d'azote liquide, du gaz de synthèse de l'ammoniac est préparé et simultanément, du monoxyde de carbone, de l'argon et du méthane sont séparés, le séparateur à froid comportant des conduits d'arrivée pour le gaz de synthèse libéré par du dioxyde de carbone, ainsi que pour de l'azote liquide et des conduits d'évacuation pour le gaz de synthèse de l'ammoniac, pour du monoxyde de carbone pur, pour un gaz combustible contenant du méthane, de l'argon et du monoxyde de carbone et pour un liquide contenant du monoxyde de carbone et de l'hydrogène.

**2.** Installation selon la revendication 1, **caractérisée en ce qu'**il est prévu un compresseur C dans lequel les gaz naissant dans les réacteurs A et B sont comprimés.

**3.** Installation selon les revendications 1 ou 2, **caractérisée en ce que** le deuxième réacteur comporte une dérivation [3] à travers laquelle le mélange de gaz de synthèse préparé dans le réacteur A est conduit totalement ou partiellement à côté du réacteur B, ce qui permet de commander son taux d'oxydation, notamment la formation de dioxyde de carbone à partir de monoxyde de carbone.

**4.** Installation selon les revendications 2 et 3, **caractérisée en ce qu'**il est prévu un compresseur C permettant de régler la pression gazeuse à une valeur qui assure l'absorption physique ou chimique du dioxyde de carbone dans l'absorbeur D.

**5.** Installation selon les revendications 1 à 4, **caractérisée en ce que** l'absorbeur D comporte un conduit d'arrivée pour le mélange de gaz de synthèse et des conduits d'évacuation pour le gaz de synthèse du méthanol consistant en monoxyde de carbone et en hydrogène, pour du dioxyde de carbone et pour le gaz de synthèse libéré par le dioxyde de carbone, ainsi que pour des gaz adaptés à d'autres synthèses, de composition adéquate.

**6.** Procédé de production simultanée de gaz de synthèse du méthanol, de gaz de synthèse de l'ammoniac, de monoxyde de carbone et de dioxyde de carbone à partir de gaz naturel, par addition de vapeur d'eau et d'oxygène dans une installation selon les revendications 1 à 5, **caractérisé en ce que** les étapes suivantes sont réalisées :

a) oxydation catalytique partielle du gaz naturel dans un premier réacteur avec des conduits d'arrivée pour du gaz naturel, de la vapeur d'eau et de l'oxygène, le gaz naturel étant transformé dans le réacteur A, avec alimentation d'oxygène en un mélange de gaz de synthèse de monoxyde de carbone, de dioxyde de carbone, d'hydrogène et de vapeur d'eau, et dans le premier réacteur, le rapport molaire de la vapeur d'eau aux hydrocarbures non oxydés étant de 1,5 à 3,0, de préférence de 1,7,
b) commande de la transformation de monoxyde de carbone en dioxyde de carbone dans un deuxième réacteur B,
c) absorption de dioxyde de carbone et préparation du mélange monoxyde de carbone/hydrogène utilisé pour la synthèse du méthanol,
d) préparation de gaz de synthèse de l'ammoniac et séparation simultanée de monoxyde de carbone, d'argon et de méthane par alimentation d'azote liquide et par un séparateur à froid E, le séparateur à froid E comportant des conduits d'arrivée pour le gaz de synthèse libéré par du dioxyde de carbone, ainsi que pour de l'azote liquide et des conduits d'évacuation pour le gaz de synthèse de l'ammoniac, pour du monoxyde de carbone pur, pour un gaz combustible contenant du méthane, de l'argon et du monoxyde de carbone et pour un liquide contenant du monoxyde de carbone et de l'hydrogène.

**7.** Procédé selon la revendication 6, **caractérisé en ce que** dans le premier réacteur, le rapport molaire de l'oxygène aux hydrocarbures non oxydés est de 0,45 à 0,7, de préférence de 0,52.

**8.** Procédé selon les revendications 6 et 7, **caractérisé en ce que** pour la transformation du gaz naturel en un gaz de synthèse, il est utilisé en catalyseur contenant de l'oxyde de nickel.

**9.** Procédé selon les revendications 6 à 8, **caractérisé en ce que** pour la transformation du gaz naturel en un gaz de synthèse selon les revendications 8 à 10, une pression de 25 à 100 bar, de préférence une pression d'environ 40 bar est utilisée.

**10.** Procédé selon les revendications 6 à 9, **caractérisé en ce que** pour l'oxydation du monoxyde de carbone en dioxyde de carbone dans le deuxième réacteur B, un procédé en une phase ou en deux phases, avec un refroidissement intercalé en présence de catalyseurs haute température est réalisé.

**11.** Procédé selon les revendications 6 à 10, **caractérisé en ce que** le gaz de synthèse généré dans les réacteurs A et B est réglé par un compresseur C à une pression de 60 à 100 bar, de préférence à une pression de 80 bar.

**12.** Procédé selon les revendications 6 à 11, **caractérisé en ce que** dans l'absorbeur D, le dioxyde de carbone est séparé du gaz de synthèse en une ou en plusieurs étapes de procédé, par voie chimique ou physique.

**13.** Procédé selon les revendications 6 à 12, **caractérisé en ce que** le gaz de synthèse est refroidi dans le séparateur

par introduction d'azote liquide à des températures de -150°C à -200°C, ce qui permet de séparer du méthane, de l'argon et du monoxyde de carbone et **en ce que** le gaz d'hydrogène résiduel est mélangé à de l'azote, selon un rapport molaire de 3 : 1 pour la préparation de gaz de synthèse de l'ammoniac.

## Tabelle I

| Gasstrom | 1 | 2 | 3 | 4 | 5 | 6 | 7 | 8 | 9 |
|---|---|---|---|---|---|---|---|---|---|
| Zusammensetzung (% Vol) | | | | | | | | | |
| Methan | 95.4 | 1.64 | 1.64 | 2.39 | 2.39 | 2.41 | 2.47 | 5.67 | 2.89 |
| Ethan | 3.91 | | | | | | | | |
| Propan | 0.03 | | | | | | | | |
| Kohlenmonoxid | | 13.88 | 13.88 | 17.71 | 17.72 | 19.32 | 19.73 | 85.41 | 29.96 |
| Kohlendioxid | 0.59 | 6.65 | 6.65 | 12.18 | 12.19 | 2.24 | | | 1.91 |
| Argon | | 0.06 | 0.06 | 0.08 | 0.08 | 0.09 | 0.09 | 0.40 | 0.14 |
| Wasserstoff | | 44.56 | 44.56 | 67.42 | 67.47 | 75.95 | 77.71 | 8.52 | 65.97 |
| Stickstoff | 0.08 | 0.02 | 0.02 | 0.02 | 0.02 | | | | |
| Sauerstoff | | | | | | | | | |
| Wasser | | 33.18 | 33.18 | 0.19 | 0.11 | | | | |
| Temp. (°C) | 22 | 412 | 412 | 40 | 41 | 36 | 36 | 36 | 36 |
| Druck (bar abs) | 23 | 39.6 | 39.6 | 37.2 | 79.3 | 77.8 | 77.8 | 76.3 | 76.3 |
| Durchfluss-menge (t/h) | 207 | 820 | 668 | 483 | 482 | 121 | 147 | 63 | 185 |

## Tabelle I (Fortsetzung)

| Gasstrom | 10 | 11 | 12 | 13 | 14 | 15 |
|---|---|---|---|---|---|---|
| Zusammensetzung (% Vol) | | | | | | |
| Methan | 0.57 | | | | | 0.60 |
| Ethan | | | | | | |
| Propan | | | | | | |
| Kohlenmonoxid | 98.19 | <5 ppm | | | | 0.4 |
| Kohlendioxid | | | | | | 98.5 |
| Argon | 0.36 | <150 pmm | 0.40 | 0.50 | 0.01 | |
| Wasserstoff | 0.87 | 75.00 | | | | 0.5 |
| Stickstoff | | 25.00 | 79.0 | | 99.99 | |
| Sauerstoff | | | 20.6 | 99.5 | | |
| Wasser | | | | | | |
| Temp. (°C) | 36 | 36 | 22 | 36 | 36 | 32 |
| Druck (bar abs) | 5 | 76.3 | 1.0 | 45 | 55.0 | 1.2 |
| Durchfluss-menge (t/h) | 24 | 168 | 1.058 | 208 | 154 | 192 |

Fig. 1

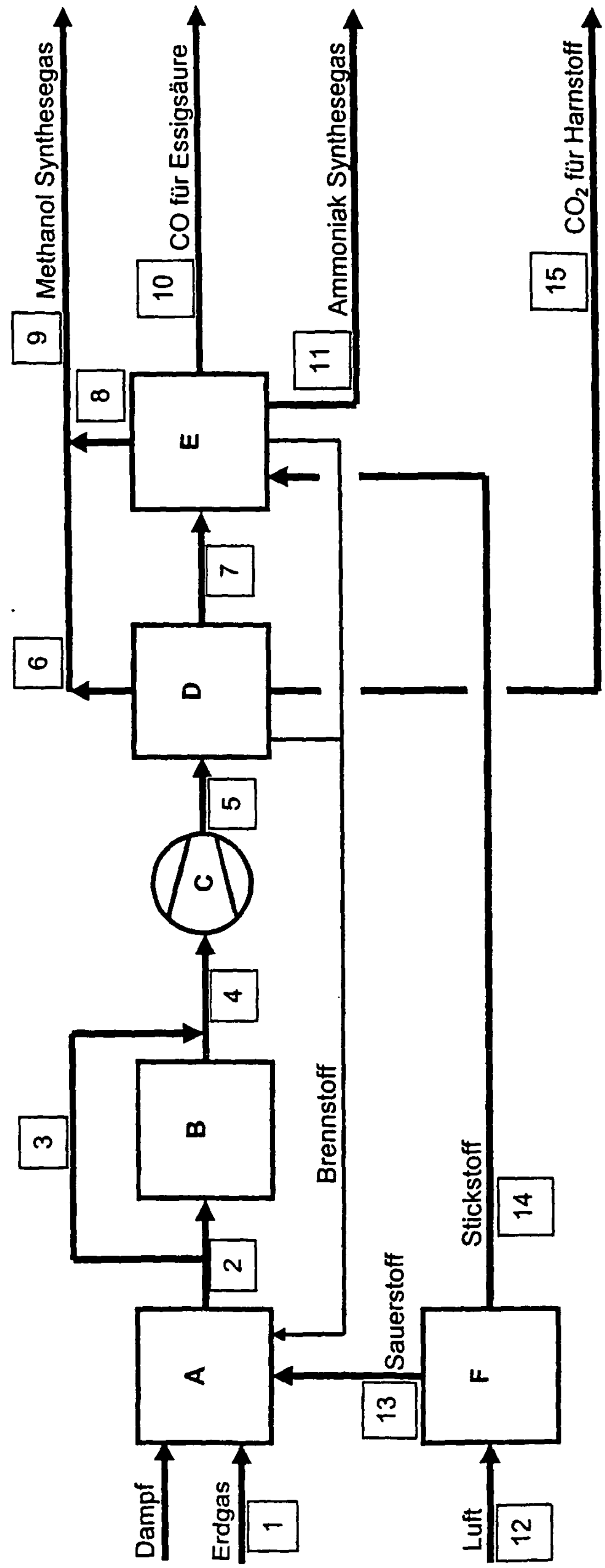
Dampf
Erdgas
1
Luft
12
A
B
C
D
E
F
2
3
4
5
6
7
8
9
10
11
13
14
15
Sauerstoff
Stickstoff
Brennstoff
Methanol Synthesegas
CO für Essigsäure
Ammoniak Synthesegas
$CO_2$ für Harnstoff

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE OS3336649 A
- JP 2000063115 B
- EP 0853608 A
- DE OS3345064 A
- EP 0999178 A
- DE 10226210 A1


### In der Beschreibung aufgeführte Nicht-Patentliteratur


- **HERMANN GÖHNA.** Concepts for Modern Methanol Plants. *Proceedings of the 1997 World Methanol Conference,* Dezember 1997
- Gas Production. Ullmans's Encyclopedia of Industrial Chemistry. VCH Verlagsgesellschaft mbh, 1989, vol. A12
- **MAX APP.** Ammonia, Methanol, Hydrogen, Carbon Monoxide, Modem Production Technologies. British Sulphur Publishing - a Division of CRU Publishing Ltd, 1997
- **EMIL SUPP.** How to produce Methanol from Coal. Springer-Verlag, 1990
- **W L E DAVEY.** *Cold Box for the Production of Multiple Products from a Stream of Syngas,* 2002, L1, 13